(19) Europäisches Patentamt
European Patent Office
Office européen des brevets

(11) Publication number: 0 227 073
A2

(12) # EUROPEAN PATENT APPLICATION

(21) Application number: 86117791.3

(22) Date of filing: 19.12.86

(51) Int. Cl.⁴: C 12 Q 1/00
C 12 Q 1/26, C 12 Q 1/28
C 12 Q 1/52, C 12 Q 1/54
C 12 Q 1/58

(30) Priority: 23.12.85 CH 5502/85

(43) Date of publication of application:
01.07.87 Bulletin 87/27

(84) Designated Contracting States:
AT BE CH DE ES FR GB IT LI NL SE

(71) Applicant: F. HOFFMANN-LA ROCHE & CO.
Aktiengesellschaft

CH-4002 Basel(CH)

(72) Inventor: Brochot, Jean
L'Agnellu Feigères
F-74160 St. Julien-en Genevois(FR)

(72) Inventor: Siddiqi, Iqbal
5 Tour de Champel
CH-1206 Geneva(CH)

(74) Representative: Buntz, Gerhard et al,
Grenzacherstrasse 124 Postfach 3255
CH-4002 Basel(CH)

(54) **Method of determining a co-enzyme.**

(57) The method makes use of the action of the co-enzyme in the production of $H_2O_2$ associated with the reaction of a hydroxylase with a decoupling agent in the presence of air or oxygen, the $H_2O_2$ being subsequently determined by quantitative breaking of the C-F bond of a fluorinated compound in the presence of peroxidase, followed by electrometric titration of the resulting ions.

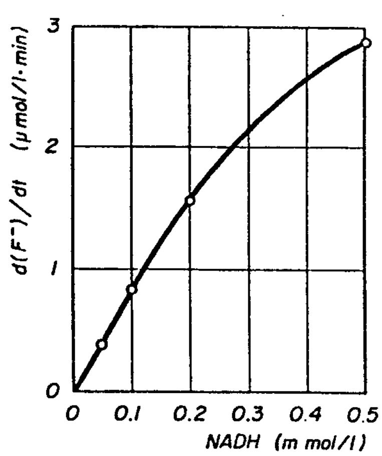


FIG. 1

EP 0 227 073 A2

Croydon Printing Company Ltd.

# METHOD OF DETERMINING A CO-ENZYME

The invention relates to biological analysis, more particularly to the determination of co-enzymes, inter alia NADH and NADPH.

As is known, the aforementioned abbreviations denote the following substances:

NADH = reduced form of NAD (or $NAD^+$) (nicotinamide-adenine dinucleotide) also called co-enzyme I or DPN (diphosphopyridine nucleotide)

NADPH = reduced form of NADP (or $NADP^+$) (nicotinamide-adenine-dinucleotide phosphate) also called co-enzyme II or TPN (triphosphopyridine nucleotide).

The reversible conversion of NAD into NADH is shown diagrammatically as follows (where R denotes a ribose-diphosphate-ribose-adenine chain):

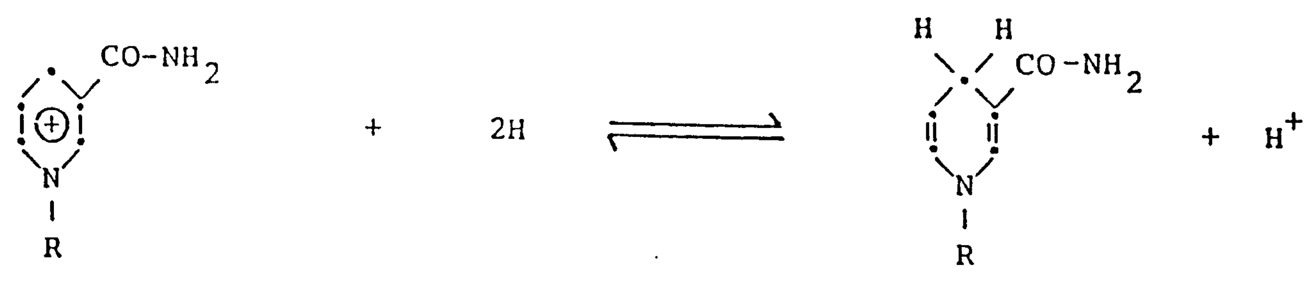


$NAD^+$ NADH

The aforementioned enzymes play a part in a large number of biochemical enzymatic reactions used as clinical tests. One example of these reactions is oxidation of α-hydroxyacids into corresponding ketonic acids in the

Bu/11.12.86

presence of a suitable dehydrogenase. One example is the oxidation of lactic acid or lactates to pyruvic acid

$$CH_3\text{-CHOH-COOH} + NAD \xrightarrow[\text{dehydrogenase}]{\text{lactate}} CH_3CO\text{-COOH} + NADH$$

Similarly, glucose-6-phosphate is oxidised in the presence of NADP and glucose-6-phosphate dehydrogenase (G6PDH) to glucono-δ-lactone-6-phosphate and NADPH. Determination of NADP or NAPH in an aforementioned reaction is very important, since it can be indirectly used for determining glucose in biological fluids after they have been converted into glucose-6-phosphate in the presence of ATP (adenosine triphosphate) and hexokinase (HK).

NADH also acts as a co-enzymatic factor in the conversion of 2-oxoglutarate into L-glutamate by ammonium salts in the presence of GLDH (glutamate dehydrogenase), thus enabling the ammonium in the reaction medium to be determined by measuring the $NADH^+$ formed. This reaction can be used for determining urea in biological fluids, since urea in the presence of urease supplies $NH_3$ which occurs as the $NH_4$ ion in the aforementioned conversion.

Similarly, the determination of transaminase in blood serum which catalyzes the conversion of α-ketoglutarate into oxaloacetate (SGOT = serum glutamate oxaloacetate transaminase) is very important in clinical chemistry, since an excess of this enzyme can indicate a coronary thrombosis.

Measurement of SGOT is associated with measurement of reduction of the oxaloacetate formed during the aforementioned reaction, by NADH in the presence of malate dehydrogenase (MDH) or glutamate dehydrogenase (GLDH). The reaction diagram is as follows:

SGOT

L-aspartate + α-ketoglutarate ------> oxaloacetate + L-glutamate

NADH, MDH

malate + $NAD^+$ <-----------------------------

$H^+$

A description of other applications associated with the determination of the $NAD^+$ and NADH factors will be found in the following documents: EP-A-29 104 (MILES); FR-A-2 299 644 (AKZO).

In view of the importance of determining the aforementioned co-enzymes in one or the other of their states of oxydo-reduction, numerous techniques have been proposed for this purpose.

For example, since NAD and NADH have different absorptions in UV, one form can be determined in the presence of the other by spectrophotometry. The sensitivity of spectrophotometric determination can also be amplified by combined use of coloured redox compounds, e.g. tetrazolium compounds which form intensely coloured formazan salts in the presence of NADH or NADPH and an electron acceptor such as phenazine methosulphate. (See e.g. document EP-A-114 267). Alternatively, use may be made of fluorimetric techniques as described e.g. in document FR-A-2 266 644.

Alternatively, an electrochemical method may be used as described in document JP 56 035 50, where NADH or NADPH is oxidised with Meldola blue, after which the reduced form of the dye is electrochemically oxidized and the oxidation current is measured.

The following reaction has recently been recommended (see R. H. WHITE-STEVENS et al., J. Biol. Chem. (1972) 247, 2358; EP-A-29 104):

$$\text{NADH (or NADPH)} + O_2 \xrightarrow[\text{pseudosubstrate}]{\text{hydroxylase, } H^+} NAD^+ \text{ (or NADP)} + H_2O_2$$

The thus-liberated hydrogen peroxide is then determined by conventional means, e.g. by its action, catalyzed by peroxydase, on a redox indicator, the oxidized form of which is determined by colorimetry.

This technique is very attractive but is of use only in a colourless, optically transparent medium, which is far from being the case with most biological fluids for analysis. In operation, also, it requires the presence of a "coupler" for preventing the dyed oxidised compound being reversibly reduced by NADH in the analysis medium. In order to determine $H_2O_2$ under these conditions, it is therefore desirable to have a technique supplying identifiable products in irreversible manner. With regard to these techniques, it has recently been disclosed (see EP-A-20 623) that excellent results with regard both to sensitivity and accuracy can be obtained in the analysis of $H_2O_2$ produced by oxidation of glucose in the presence of glucose oxidase, the method consisting in reacting the $H_2O_2$ with an aromatic fluorinated compound in the presence of peroxidase so as to liberate fluoride ions, which are then electrometrically determined by using an electrode specific to these ions.

It has now been discovered that a similar technique is of use for determining NADH or NADPH co-enzymes. This technique has resulted in the method according to the invention as defined in the accompanying claim 1. Use can be made of acetate, phosphate and cacodylate buffers over a pH range of approx. 5-8 and an ionic strength of 0.1 to 1. Cacodylate buffer at pH 5.5 or 7.5 and ionic strength of 0.1 to 0.5 are preferably used.

The fluoride ion liberated by this method is preferably determined by the electrometric method as disclosed in document EP-A-20 623. However, any method based on use of another electrode selective to fluoride ions will be equally suitable.

The general principle of the present method can be briefly described as follows: The invention is based on a system whereby a buffer medium of given ionic strength and pH containing the reduced co-enzyme to be determined is mixed with an excess of a "decoupling" pseudo-substrate, e.g. sodium benzoate and the enzyme monooxygenase salicylate hydroxylase (SH) which, in the presence of NADH (or NADPH), catalyzes the reduction of the oxygen present to hydrogen peroxide. The amount of hydrogen peroxide formed is proportional to the amount of NADH for analysis. In the presence of an excess of a fluorinated aromatic compound and a peroxidation agent such as peroxidase (HRP or POD), an irreversible break occurs in the C-F bond of the fluorinated compound, with corresponding liberation of $F^-$ ions at a reaction rate proportional to the quantity of enzyme present. There is also a stoichiometric relation between the aforementioned quantity of liberated fluoride ions and the quantity of co-enzyme to be determined. Thus, "fixed-time" analyses, such as "end-point" determination, may be made by determining the reagents after a given time, always the same, in a set of similar analytical operations. In general, however, it is preferable to measure the liberation rates of $F^-$ ions under highly standardized conditions in order to ensure good reproducibility of measurements. For example, the gradients of the rate curves (which of course depend on certain reaction parameters as well as the concentration of substance to be measured) are advantageously measured after a certain latency time, which is usually kept constant during a set of comparative tests.

However, the latency time may differ from one analysis to the other.

Preferably the concentration of liberated $F^-$ ions is determined by using an electrode sensitive to $F^-$ ions but inert towards other types of ions. The preferred type of electrode is a type 96-09 electrode selective to $F^-$ ions and produced by ORION RESEARCH INC. CAMBRIDGE, MASS. USA. However, other electrodes may be equally suitable. All details regarding use of these electrodes for deternining $F^-$ ions may be found in the aforementioned document EPA-20 623.

As will be seen hereafter, the analytical method of the invention is applicable to the determination of many biological reactions and systems involving the presence of the co-enzymes in oxidized or reduced form.

The pH to be selected will depend on the species to be analyzed and the reaction media. As said before, the pH of the buffer used for carrying out the desired determination, is preferably in the range 5 to 8, better from 5.5 to 7.5 with an anionic strength from 0.1 to 0.5. These limits can however be extended below and above said range in special cases.

Thus, several experiments have been effected to determine the importance of parameter variations on the performance of the present method.

For instance the effect of pH on the rate of F-production was tested, the cacodylate anion molarity (0.1) being kept constant and the molarity of the free cacodylic acid being varied to modify the pH. In these tests, the molarity of the pseudosubstrate being 0.03, the ionic strength of the buffer was constantly 0.13. Under these conditions, the reaction rates increased with decreasing pH, this effect

being particularly significant in the upper NADH concentration ranges (see Example 2).

In another set of experiments, the total cacodylate was kept constant (0.1M) but the ratio of cacodylate anion to free cacodylic acid was varied in order to change the pH. In order to compensate the resulting changes of ionic strength and maintain a constant value of 0.13 (including 0.03 from the benzoate), either $SO_4^{--}$ or $Cl^-$ ions were added. The effects of pH (see Example 3) were of the same general trend observed in the earlier case but less significant; $Cl^-$ definitely acted as an inhibiter. Furthermore, the effects of changing the buffer ingredients concentrations (changing the ionic strength for instance) are only significant at the low pHs of the range and with relatively large amounts of NADH. For instance, increasing anionic strength with sulfate or cacodylate at pH 7 had virtually no effect (see Example 4). In this case $Cl^-$ also acted as an inhibitor.

The effect of varying the amount of peroxidase at different pHs was also tested. It was surprisingly found that, especially with relatively large concentrations of NADH ($4x10^{-4}$M), that the rate of F-liberation increased with the lowering of the amount of peroxidase used; the effect was particularly strong at pH 5.5. Simultaneously, the latency increased, i.e. the lag time between when adding the analyte to the medium and the moment the reaction starts (see Example 5).

When the present reaction medium is used for determining precursor systems, i.e. systems where the quantitative formation of the co-enzyme depends on one or more successive transformations of a substance to be measured, the technique to be applied is very similar to that described hereinbefore. The reason (and this is one of the significant advantages of the invention) is that the detection and

electrochemical measurement of fluoride ions is unaffected by the presence of numerous other factors and dissolved substances in the reaction medium. The present process is also directly useful for measuring glucose and urea by procedures similar to those mentioned in the introduction.

Glucose, for example, is determined by first converting it to glucose-6-phosphate in the presence of ATP and hexokinase (or another enzyme having similar properties). Next, since the medium contains a known quantity of NAD, the previously-mentioned process is used to measure the NADH formed during conversion of glucose-6-phosphate to glucono-δ-lactone-6-phosphate in the presence of G6PDH. At the start of the reaction, the reaction medium does not contain NADH, which appears during the enzymatic process. Actually the three reactions, i.e. the one catalyzed by hexakinase, the one catalyzed by G6PDH and the one catalyzed by hydroxylase, occur simultaneously in the presence of NADH and ATP. Accordingly, the formation of NADH is continuously measured from the beginning of the reaction and the rate constant of this reaction is the critical factor. Similar considerations apply to all other biochemical reactions involving the present co-enzymes either as the starting products or as reaction products. The determination of urea is an example.

A sample of urea taken from a biological fluid, e.g. urine or blood plasma, and mixed with a suitable buffer, is mixed with an excess of urease, oxoglutarate and GLDH accompanied by an exactly known quantity of NADH (also in excess but of the same order as the urea to be measured, to avoid problems of disproportion). Next, after waiting for a given time for transformation of NADH into $NAD^+$ to occur, the fluorinated compound, benzoate and hydroxylase are added, the $F^-$ ion electrode is inserted into the mixture and the mixture is agitated in air. A catalytic quantity of peroxidase (POD) is added and the variation in electrode

potential with time is measured in order to determine the quantity of NADH not used in the reaction and thus deduce the quantity of urea in the analyzed sample.

Document EP-A-20 623 gives details of the operating technique using the fluorine electrode and also sets out the physico-chemical considerations when interpreting the results.

In short, the liberation rate of $F^-$ ions after reaction of an unknown sample is determined preferably by referring to a calibration curve. A calibration curve can be obtained as previously described by determining a set of samples containing known concentrations of co-enzyne. The rate of liberation of $F^-$ ions is recorded for each sample and the gradient of the kinetic curves is measured at a time (the same of course for each sample) when the rate curves are almost straight. Next, the values of the gradient are graphically recorded in dependence on the concentrations of co-enzyme so as to obtain a standard reference curve. The measured electrometric parameters used for preparing the kinetic curves can be the recorded voltages of the electrometric system used in combination with the fluorine electrode (mV) or preferably the corresponding values of $[F^-]$ which can be calculated by the Nernst equation, which in the present case has the following form:

$$E = E' - S.Log [F^-]$$

where E is the measured voltage and E' is an experimentally-determined constant belonging to the system and including the activity factors and the liquid-junction potentials. S is the "Nernst gradient" and is a constant equal to about 57.5 mV (in the cacodylate buffer at pH 7.5) for a variation of 10 units in the concentration of $F^-$ ions, the concentration being expressed in mols/l. If the values of $[F^-]$ calculated from the above relation are used

in the rate graphs instead of the values in mV, the resulting curves are very close to straight lines, the gradient of which is easier to determine and results in more accurate reference graphs.

The following are preferred fluorinated aromatic compounds on the ring, suitable for working the invention: 4-fluoroaniline, 4-fluorophenol, 2,3,5,6-tetrafluorophenol and pentafluorophenol; it is preferred to use 4-fluorophenol and the aforementioned tetra- and pentafluoro derivatives.

The invention is illustrated in greater detail by the following examples, which will be more clearly understood by referring to the accompanying drawings in which:

Fig. 1 is a graph showing, at pH 7.5, the variation in the amount of NADH with the rate of liberation of $F^-$ ions;

Fig. 2 is a graph similar to Fig. 1 but relating to analysis of SGOT;

Fig. 3 is a graph similar to Fig. 1 but relating to analysis of glucose;

Fig. 4 is a graph similar to Fig. 1 but relating to analysis of urea;

Fig. 5 is a graph showing the variation of the rate of liberation of $F^-$ ions with the pH for several concentration of NADH, and

Fig. 6 is a graph similar to that of Fig. 1 but run at pH 5.5.

EXAMPLE I

Determination of NADH

A buffer solution was used for this determination according to the invention. Conventional cacodylate buffer at pH 7.5 was used. 21.4 g of sodium dimethylarsinate trihydrate (MERCK) were dissolved in 800 ml of twice--distilled water and 2 ml of a $10^{-3}$ M solution of NaF were

added (traces of NaF are added to reaction mediums in order to stabilize the base potential of the fluorine electrode). The pH was adjusted to 7.5 (1 N HCl) and the mixture was made up to 1 litre with twice-distilled $H_2O$.

Reaction medium:

The operating medium in the aforementioned buffer was prepared by dissolving the following reagents in the following molar concentrations: 0.01 M p-fluorophenol; 0.03 M sodium benzoate; 2 U/ml horse-radish peroxidase (HRP); 0.11 U/ml salicylate hydroxylase, and 3 µM NaF.

Standard solution of NADH:

In the aforementioned cacodylate buffer, a stock solution of $5 \times 10^{-2}$ M NADH was prepared by dilution of the commercial reagent (SIGMA). Next, using the same buffer, the following standard solutions were prepared by dilution: $2.5 \times 10^{-2}$ M; $10^{-2}$ M; $5 \times 10^{-3}$ M and $2 \times 10^{-3}$ M.

Method of operation:

4.9 ml of the aforementioned reaction medium were poured into a 10 ml polyethylene beaker and an electrode specifically sensitive to $F^-$ ions (type Orion 96-09, ORION RESEARCH CO., Cambridge, Mass.) comprising an internal reference electrode was immersed in the liquid with magnetic agitation. The electrode was connected to a very sensitive commercial electrometer (KEITHLEY ELECTROMETER, type 619, Cleveland, Ohio).

The device was switched on and left to stabilize for 3 minutes at ambient temperature, after which 0.1 ml of one of the aforementioned standard solutions was added and the liberation rate of $F^-$ ions by the aforementioned reaction

was measured for about 2 minutes by recording the corresponding electrode potential. The recorded data were also transmitted to a computer which supplied the gradient of the rate curve in its linear region (after about 20 seconds and for 30 seconds to 1 minute), these parameters of course were kept constant during the entire calibration phase and for subsequent analysis of unknown samples, which was done in identical manner.

The results are shown graphically in Fig. 1. The graph shows the variation in liberation rates of $F^-$ ions in µmols/l/min (µmols/l.min) in dependence on the molar concentration of NADH in the analyzed medium.

Unknown solutions of NADH were analyzed in identical manner and the molar concentration of NADH in the operating medium was determined by comparing the measured kinetic value with the graph in Fig. 1.

## Example 2

### Effect of pH on NADH determination

These experiments were run using the procedure disclosed in Example 1. The buffer (a) was prepared by dissolving cacodylic acid (dimethyl-awinic acid) in bidistilled water and adding N sodium hydroxide to adjust the pH; then the required quantities of the following reagents were added to obtain the required molar concentration; NaF $10^{-5}$M; 4-fluorophenol $10^{-2}$M; Na benzoate $3x10^{-2}$M. The concentrations of cacodylic acid (total, free acid and anion) are given below as a function of the pH of the buffer selected. The anionic strength was kept constant (0.13).

| Ingredients | pH | | | | |
|---|---|---|---|---|---|
| | 5.5 | 6.0 | 6.5 | 7.0 | 7.5 |
| Cacodylic acid (total) | .556 | .244 | .146 | .114 | .105 |
| (free acid) | .446 | .144 | .046 | .014 | .005 |
| (anion) | .1 | .1 | .1 | .1 | .1 |

The enzyme solution (b) contains 50 U/ml of peroxidase and 5 U/ml of salicylate hydroxylase.

The NADH standards (C, $5x10^{-3}M$ to $2x10^{-2}M$) are prepared as in Example 1 in diluted buffer (0.0M) at pH 7.5.

For carrying out the analysis, 4.8 ml of (a) and 0.1 ml of (b) were introduced into a 10 ml polyethylene beaker. The fluoride sensitive electrode was immersed into the liquid and, after 2 min, 0.1 ml of (c) was added. The kinetic curves were recorded as usual and the slope was calculated by regression analysis of the values taken from the most characteristic portion. It should be noted that the time at which this characteristic portion of the curve develops varies with pH, the latency increasing with lower pHs. Fig. 5 illustrates diagrammatically the variation of the rate (µmole $F^-$/l/min) as a function of NADH concentration (in µmoles in the samples) for several pH values. Note the inverse curvature of the pH 5.5 curve.

The results are also reported below in table form, rates in µmoles of $F^-$/l/min being expressed as a function of pH and NADH molar concentration in the standards (c).

| | NADH concentration ($x10^{-4}M$) | | | |
|---|---|---|---|---|
| pH | 1 | 1.6 | 2 | 4 |
| 7.5 | 3.38 | 4.76 | 5.81 | 8.98 |
| 7.0 | 4.0 | 5.62 | 6.67 | 10.58 |
| 6.5 | 4.36 | 6.30 | 7.37 | 11.54 |
| 6.0 | 4.25 | 6.10 | 7.51 | 13.34 |
| 5.5 | 4.17 | 6.95 | 8.95 | 24.95 |

In view of the above results a typical NADH calibration curve at pH 5.5 was run using a 0.1M cacodylic buffer adjusted at pH 5.5 with NaOH N. The other components were like above except NaF $5x10^{-6}$M.

The enzyme solution contained 25 U/ml of peroxidase and 5.5 U/ml of salicylate hydroxylase.

The following NADH solutions ($10^{-4}$M multiples) were tested (between brackets, the corresponding rates observed): 0.5(0.58); 1(1.27); 2(3.54); 3(7.3); 4(16.5); 5(26.03). The curve expressing these results (in µmole of $F^-$/min) is represented in Fig. 6.

## Example 3

Effect of pH on NADH determination, keeping the concentra- ation of cacodylic acid constant.

In further experiments, the pH of the buffer was varied but keeping constant (0.1M) the total amount of the cacodylic species, i.e. the ratio of free acid to cacodylate ion was varied as shown below. In order to keep the ionic strength constant (0.13), 0.1 being contributed by cacodylate and other salts (0.03 being due to the benzoate), sodium sulfate (or sodium chloride) were added. The respective quantities of these ingredients are listed below. The other ingredients NaF and p-fluorophenol were as previously described [Example 2,(a)].

The enzyme solution contained 30 U/ml of peroxidase and 5 U/ml of salicylate hydroxylase.

The NADH standards (c) were $10^{-2}$N and $4x10^{-3}$M. Thus 0.15 ml samples gave $3x10^{-4}$ amd $1.2x10^{-4}$ moles, respecively.

| Ingredients | pH 5.5 | 6.0 | 6.5 | 7.0 |
|---|---|---|---|---|
| cacodylic (mole/mole) cacodylate | 4.6 | 1.44 | 0.46 | 0.144 |
| NaCl (molar) | .082 | .059 | .0314 | .0126 |
| $Na_2SO_4$ (molar) | .0273 | .0197 | .0105 | .0042 |

The analysis was run as previously described using 4.65 ml of buffer, 0.2 ml of the enzyme solution (b) and 0.15 ml of the NADH standard (c).

The results are given in the table below, the first rate values in µmoles/l/min being for the $1.2x10^{-4}$ mole aliquot and the second for $3x10^{-4}$ mole.

| Assay | pH 5.5 | 6.0 | 6.5 | 7.0 |
|---|---|---|---|---|
| NaCl | 1.74(3.63) | - | - | 2.43(4.88) |
| $Na_2SO_4$ | 4.0 (11.5) | 4.71(9.86) | 4.87(8.85) | 4.04(7.97) |

These results show that the effect of pH was only significant for the higher concentration of NADH and that chloride ion acts as an inhibitor toward salicylate hydroxylase.

## Example 4

Effect of changing the concentration of the buffer (ionic strength) on NADH determination.

The concentration of ingredients in the reaction buffer was varied but the pH (7) was kept constant. The buffer was based on cacodylate with or without additional salts (NaCl;

$Na_2SO_4$); it was $5x10^{-6}$M in NaF; the other ingredients, 4-fluorophenol and sodium benzoate were as disclosed in the foregoing Examples. Three cases were studied with ionic strength 0.13, 0.23 and 0.43, respectively (the contribution of the benzoate to the ionic strength was 0.03).

The concentration of the buffer ingredients is given below for each three cases. Note that either the NaCl or the $Na_2SO_4$ was used as an additional salt. All the concentrations are molar. The figures between brackets refer to the cases when no additional salts were used.

| Ingredients | Ionic Strength 0.130 | 0.230 | 0.430 |
|---|---|---|---|
| Cacodylate | | | |
| (Total) | 0.1 (0.114) | 0.1 (0.227) | 0.1 (0.452) |
| (free acid) | .0126 (.014) | .012 (.027) | .0116 (.052) |
| (ionic) | .0874 (.1) | .088 (.2) | .0884 (.4) |
| NaCl | .0126 | .112 | .3116 |
| $Na_2SO_4$ | .0042 | .0373 | .1039 |

The enzyme solution contained 80 U/ml of peroxydase and 8 U/ml of salicylate hydroxylase.

Two NADH standard solutions were used: $3.2 \times 10^{-2}$ M and $0.8 \times 10^{-2}$ M.

The assay procedure was run as described in the previous examples with 3.9 ml of buffer, 0.05 ml of the enzyme solution and 0.05 ml of the NADH standard. The rate results recorded as explain previously are given in the next table in µmole $F^-$/min against concentration, i.e. the first figure refers to an aliquot of $10^{-4}$ mole and the

second, between brackets, to 4 x $10^{-4}$ mole.

| Assay | Ionic Strength | | |
|---|---|---|---|
| | 0.130 | 0.230 | 0.430 |
| Cacodylate only | 1.3 (4.45) | 1.8 (4.74) | 1.66 (4.92) |
| NaCl | 1.61 (4.42) | 0.37 (1.17) | 0.2 (0.59) |
| $Na_2SO_4$ | 1.35 (4.02) | 1.43 (4.70) | 1.57 (4.43) |

The above results show that NADH is not much dependent on ionic strength in the claimed range except when chloride is used. Therefore chloride can be used to adjust the ionic strength in cases when the rates are too high and should be lowered.

## Example 5

### Effect of peroxidase concentration on the rate of $F^-$ liberation

Three buffers were prepared to provide pH's of 5.5, 6.5 and 7.5, respectively. Two were cacodylate-based, the third was a "Tris" buffer. In addition to the usual 0.03 M benzoate, NaF $10^{-5}$ M and $10^{-2}$ M p-fluorophenol, the buffers contained the ingredients listed below (all concentrations or molar).

| Ingredients | pH 5.5 | 6.5 | 7.5 |
|---|---|---|---|
| Cacodylic acid | .082 | .0314 | - |
| Cacodylate ion | .018 | .0686 | - |

| | | | |
|---|---|---|---|
| $Na_2SO_4$ | .0273 | .0105 | .006 |
| Tris base | - | - | .0176 |
| Tris $NH_3^+$ | - | - | .0824 |
| Ionic strength | 0.13 | 0.13 | 0.17 |

Two separate enzyme solutions were prepared

(b1): 10 U/ml salicylate hydroxylase

(b2): three standard peroxidase solutions, respectively 500 U/ml, 100 U/ml and 20 U/ml

Two solutions (c) of NADH (4 x $10^{-2}$ M and $10^{-2}$ M) were also used in this test.

The tests were carried out as usual with 4.8 ml of buffer, 0.05 ml of (b1), 0.05 ml of (b2) and, after two min. of stabilization, 0.05 ml of (c).

The results are given below in µm $F^-$/min; the first table concerns the lower concentration of NADH ($10^{-4}$ M standard); the second table concernes the 4 x $10^{-4}$ M standard of NADH. The values in bracket are the lag time in seconds separating the start of the reaction from the addition of the NADH sample. Note the relative long latency period corresponding to low peroxidase concentrations; this is compensated by unusually high reaction rates.

| | pH | | |
|---|---|---|---|
| Assay ($10^{-4}$ M NADH | 5.5 | 6.5 | 7.5 |
| Peroxidase | | | |
| 5 U/ml | 0.72 (10) | - | 0.79 (5) |
| 1 U/ml | 1.36 (75) | - | 1.77 (25) |
| 0.2 U/ml | 2.10 (250) | - | 1.86 (40) |

| | | | |
|---|---|---|---|
| (4 x $10^{-4}$ NADH) | | | |
| Peroxidase | | | |
| 5 U/ml | 4.5 (10) | 3.35 (10) | 2.98 (5) |
| 1 U/ml | 7.5 (80) | 4.53 (25) | 4.06 (10) |
| 0.2 U/ml | 42.0 (275) | 4.65 (75) | 4.25 (25) |

## Example 6

In this example a comparison is made between the rate results measured in Example 5 and the corresponding end-point titration derived by integration of the rate curve at the levelling point. In the following table the first result is the rate in µmole $F^-$/min and the second result (in bracket) is the total amount of fluoride liberated (in µmole). These results show that in most cases a good correlation exists and that both techniques are usable in some cases for NADH measurements.

| Assay ($10^{-4}$ M NADH) | pH 5.5 | pH 6.5 | pH 7.5 |
|---|---|---|---|
| Peroxidase | | | |
| 5 U/ml | 0.72 (0.9) | - | 0.79 (0.5) |
| 1 U/ml | 1.36 (2.3) | - | 1.77 (2.4) |
| 0.2 U/ml | 2.10 (5.4) | - | 1.86 (3.3) |
| (4x$10^{-4}$ M NADH) | | | |
| Peroxidase | | | |
| 5 U/ml | 4.5 (4.5) | 3.35 (4.6) | 2.98 (4.4) |
| 1 U/ml | 7.5 (10.8) | 4.53 (7.4) | 4.06 (7.3) |
| 0.5 U/ml | 42 (38.9) | 4.65 (10.1) | 4.25 (9.0) |

## EXAMPLE 7

Analysis of SGOT

Reagent A:

A solution was prepared in the cacodylate buffer described in example 1 (pH 7.5) using the following ingredients in the following molar concentrations: 4-fluorophenol 0.012 M (1.345 g/l); sodium benzoate 0.037 M (5.33 g/l); L-aspartic acid 0.26 M (34.609 g/l).

Reaction medium:

This solution was prepared by using reagent A as a solvent and a solution of NADH in the aforementioned 0.1 M cacodylate buffer (NADH 50 mM, (38 g/l). Use was also made of reagents from the ROCHE (DIAGNOSTICA) kit present in the form of portions each containing malate dehydrogenase (MDH) enzyne and lactate dehydrogenase (LDH) enzyme and a small proportion of NADH. This kit, called "GOT OPT., DGKC", is described in the corresponding operating instructions (List No. 07 14410 [10 x 10 ml]) and contains bottles holding the following portions: NADH 2.3 mol, LDH 0.26 µkat = 15.6U, MDH 0.13 µkat = 7.8 U (1 µkat = 60 U). The presence of LDH is due to the fact that the kit is intended for analysis of SGOT in serum. Serum contains pyruvate which has to be enzymatically decomposed beforehand so that it does not interfere with the measurement of SGOT.

In order to prepare the present solution, 1.9 µmols of NADH, i.e. 40 µl of the aforementioned 50 mM NADH solution, were added to the contents of a bottle in the kit and the mixture was dissolved in 10 ml with reagent A. The solution was therefore 0.42 mM NADH and contained 1600 U LDH and 800 U MDH per litre.

Standard samples of SGOT

The reagent used was GOT-CALBIOCHEM, 2180 IU/ml, comprising a 3.2 M solution of ammonium sulphate, 2.5 mM of 2-oxoglutarate and 50 mM of sodium malate. The reagent was first diluted at 1/150 with water, and the dilution was re-diluted at 1/97 with the aforementioned cacodylate buffer. The resulting solution contained 0.15 U/ml and was used to prepare the following standards: (b) 30 U/l; (c) 60 U/l; (d) 90 U/l. A control (a) without SGOT was also prepared by correspondingly diluting a solution of 3.2 M $(NH_4)_2SO_4$, 2.5 mM 2-oxoglutarate and 50 mM sodium malate.

Reagent B:

This solution was prepared from 0.1 M cacodylate buffer and contained 6 U of salicylate hydroxylase (SH) and 100 U of HRP per ml.

Method of operation:

The procedure was as in Example 1. 3.2 ml of reaction solution, 0.6 ml SGOT standard and 0.1 ml of an aqueous 468 mM solution of 2-oxoglutarate were pipetted into a 10-ml beaker. The reaction was then allowed to occur for 12 minutes under agitation at ambient temperaLure, after which the fluoride electrode was immersed therein. The mixture was left to stabilize for exactly 3 minutes and 0.1 ml of reagent B was added, resulting in fornation of $H_2O_2$ in proportion to the NADH remaining to be deternined, and the liberation rate of $F^-$ was measured. The results are given in a graph in Fig. 2, showing the decrease in the $F^-$ liberation rate (in µmols/l.min of $F^-$ ) with respect to the increase in SGOT in the titrated sample. The resulting curve was of subsequent use in

determining unknown samples of SGOT, inter alia blood serum.

EXAMPLE 8

Determination of glucose

The following reagents and solutions were prepared:

A: Cacodylate buffer:

Sodium benzoate 0.03 M (216 mg/50 ml); 4-fluorophenol 10 mM (56 mg/50 ml); NaF $3 \times 10^{-6}$ M (0.15 ml of $10^{-3}$ M/50 ml sol); twice-distilled water.

B: Reaction medium:

The contents of a bottle of "Reagent 1" from the "Glucose Rapid Test (ROCHE)" glucose-measuring kit, list No. 07 1100 4, was dissolved in 24.5 ml of buffer A. The bottle contained the following products:

| | |
|---|---|
| ATP (adenosine triphosphate) | 50 µmol; |
| $NAD^+$ | 50 µmol |
| HK (hexokinase) | >7 U |
| G6-PDH (Glucose-6-phosphate dehydrogenase) | 8 U. |

The resulting solution had the following molar concentration:
ATP $2.04 \times 10^{-3}$ M; $NAD^+$ $2.04 \times 10^{-3}$ M; HK 0.286 U/ml; G6-PDH 0.326 U/ml.

C: The following enzymes were dissolved in the following proportions in buffer A: POD (peroxidase) 0.4 mg/ml (100 U/ml); salicylate hydroxylase (SH) 2.5 mg/ml (5.75 U/ml).

D: Standard glucose solutions: in a 0.1% solution of benzoic acid, standard glucose solutions were prepared at the following concentrations in g/l: 50, 100, 200, 300 and 400.

Method of operation:

The method was similar to that described in the previous Examples except that the present analysis consisted in determining NADH as soon as formed by reduction of $NAD^+$. Consequently, there was no waiting period, unlike the previous cases.

3.8 ml of reaction solution B and 0.1 ml of solution C were used. The electrode was immersed in the medium and, after stabilization for 3 minutes, 0.1 ml of the standard glucose solution was added and measurements were made for 1 to 2 minutes.

The gradient of the rate curves as obtained by the computer is shown hereinafter in dependence on the concentration of the standard glucose solutions:

| Glucose solution (g)l | Speed (µmol $F^-$/l.min) |
|---|---|
| 0 | 0.074 |
| 0.5 | 0.361 |
| 1 | 0.610 |
| 2 | 0.963 |
| 3 | 1.176 |
| 4 | 1.328 |

These values are shown in the graph in Fig. 3, which was then used as a reference curve for determining unknown glucose solutions.

EXAMPLE 9

Determination of urea

The following solutions and reagents were prepared:

A: Cacodylate buffer: identical with that in Example 8.

B: Reaction medium: use was made of the reagents supplied by Roche in the urea UV test (ROCHE) kit, list No. 07 13228. Reagent (1) contains the following per portion: urease >25 U; 2-oxoglutarate 197 µmols; NADH 6 µmols. Two of the aforementioned portions were dissolved in 50 ml of buffer A, after which 200 µl were added from the glutamate dehydrogenase solution (about 60 U GLDH) from portion 3 in the ROCHE kit and 250 µl of a $5 \times 10^{-2}$ M NADH solution in buffer A (38 mg/ml).

C: Enzyme solution: this contained 0.4 mg/ml POD and 2.5 mg/ml SH in buffer A.

D: Standard urea sample:

These samples were obtained by diluting a 7.13 mM stock solution in water.

The following samples (0.2 ml) were prepared as follows:

0.04 ml stock + 0.16 ml $H_2O$ : 1.43 mmols
0.08 ml stock + 0.12 ml $H_2O$ : 2.85 mmols
0.12 ml stock + 0.08 ml $H_2O$ : 4.28 mmols
0.2 ml stock : 0.0

Method of operation:

The procedure was as follows:

3.7 ml of reagent solution B followed by 0.2 ml of urea sample were poured into a mechanically agitated beaker. The reaction was then allowed to continue for exactly 12 minutes, after which the fluorine electrode was immersed and, after 3 minutes of stabilization, 0.1 ml of solution C was added. The liberation rate of $F^-$ was then measured for 1 to 2 minutes and the gradient of the rate curve was determined. The results are shown hereinafter in Table form, and also in Fig. 4.

| Urea Sample | Speed (µmols $F^-$/l. min) |
|---|---|
| 0 (control) | 1.32 |
| 1.43 | 1.18 |
| 2.85 | 0.99 |
| 4.28 | 0.76 |
| 7.13 | 0.22 |

The resulting values were then used to measure the urea content of unknown samples, by comparison.

## CLAIMS

1. A method of determining a co-enzyme, inter alia NADH or NAD(P)H present in a biochemical analysis system containing this co-enzyme either in reduced form, in which case the excess thereof is determined after a given time, or in oxidized form, in which case the reduced form is determined at the same rate as it is formed in the system, characterised in that the NADH or NAD(P)H to be determined is oxidized by air or oxygen in a buffer medium at controlled pH and ionic strength in the presence of monoxygenase salicylate hydroxylase enzyme and a decoupling agent, inter alia benzoate, resulting in the formation of $H_2O_2$ in proportion to the quantity of the aforementioned co-enzyme present, the $H_2O_2$ is reacted with a fluoroaromatic compound in the presence of a peroxidation agent so that the compound is catalytically and irreversibly oxidized, breaking the C-F bond and forming $F^-$ ions, and the ions are electrometrically titrated, using an electrode selectively sensitive to $F^-$ ions.

2. A method according to claim 1, characterised in that the buffer is phophate, acetate or cacodylate at pH 5-8 and ionic strength 0.1 to 0.5.

3. A method according to claim 2, characterised in that the buffer is 0.1 to 0.5 M cacodylate at pH 5.5 to 7.5.

4. A method according to claim 2, characterized in that the buffer also contain $SO_4^{--}$ ions.

5. A method according to claim 1, characterized in that the titration of the $F^-$ion involves measuring the rate of formation of said ion per unit time.

6. A method according to claim 1, characterized in that the titration of the $F^-$ ion involves measuring the concentration of said ions after a given time.

7. A method according to claim 1, characterised in that it is used for determining "Serum Glutarate Oxalacetate Transaminase" SGOT, and the oxalacetate enzymatically formed by reaction of L-aspartate with α-ketoglutarate is converted into malate by reduction with NADH, the excess whereof is then determined after a given time.

8. A method according to claim 1, characterised in that it is applied to determination of glucose after it has previously been converted into glucose-6-phosphate in the presence of ATP and hexokinase, whereby glucose-6-phosphate is oxidized in the presence of $NAD^+$ and G6PDH into glucono-δ-lactone-6-phosphate with formation of NADH, which is then determined as soon as formed.

9. A method according to claim 1, characterised in that it is used for determination of urea previously converted into ammonium salts by urease, whereby ammonium 2-oxoglutarate is converted into L-glutamate in the presence of GLDH and NADH, and the excess NADH is then determined after a given time.

***

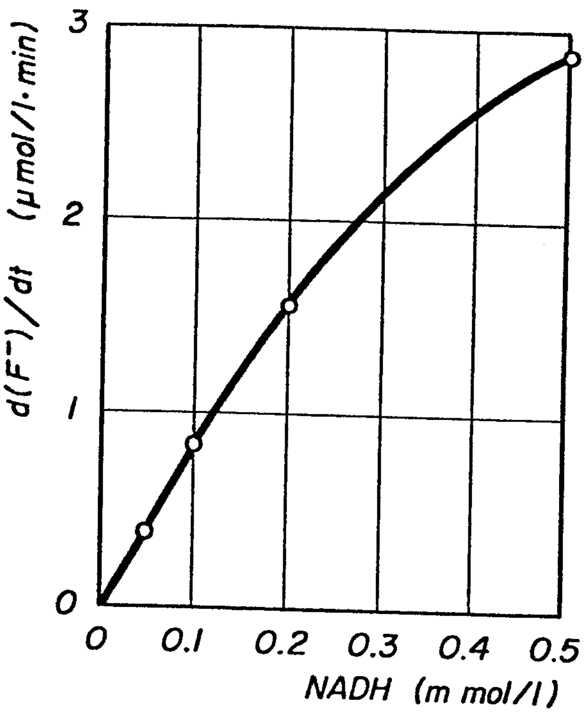


FIG. 1

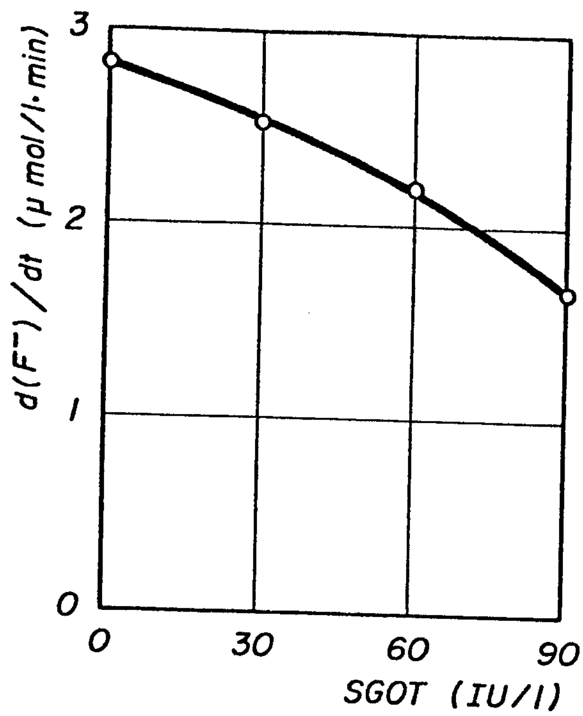


FIG. 2

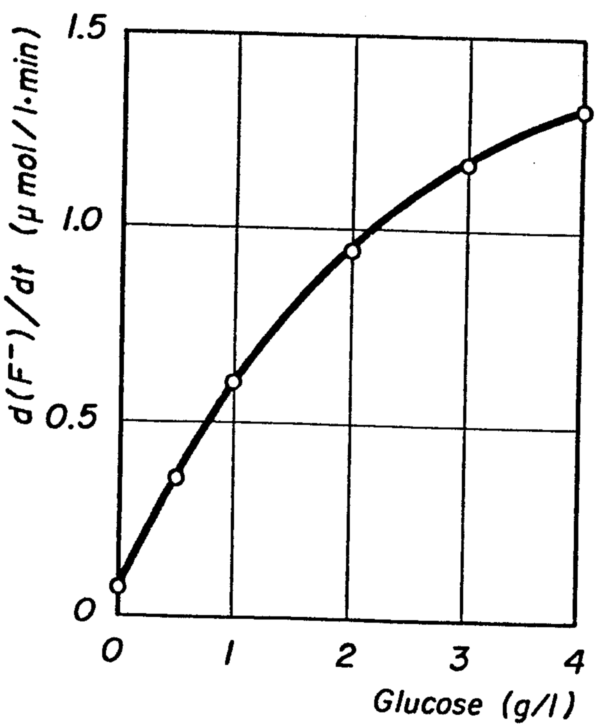


FIG. 3

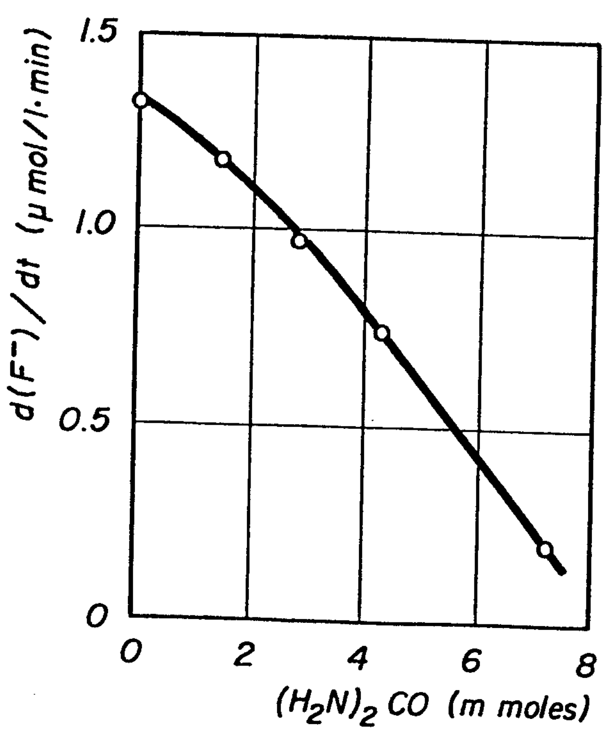


FIG. 4

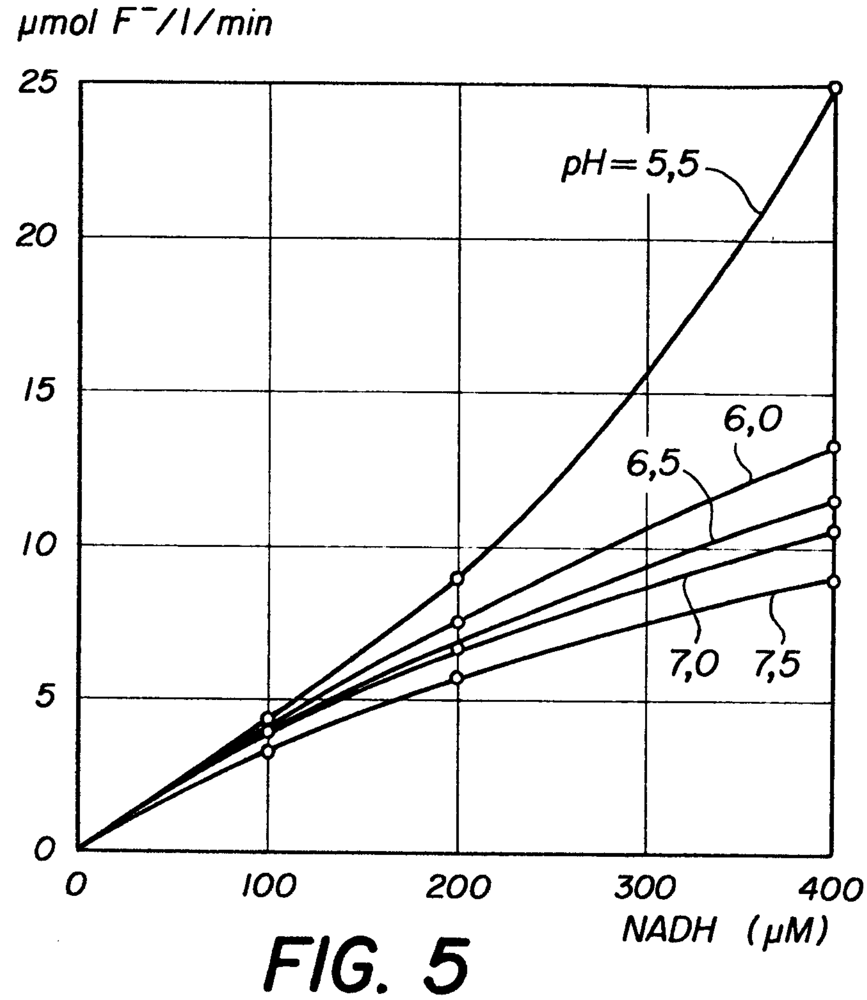
µmol F⁻/l/min
25
20
15
10
5
0
0
100
200
300
400
NADH (µM)
pH=5,5
6,0
6,5
7,0
7,5

FIG. 5

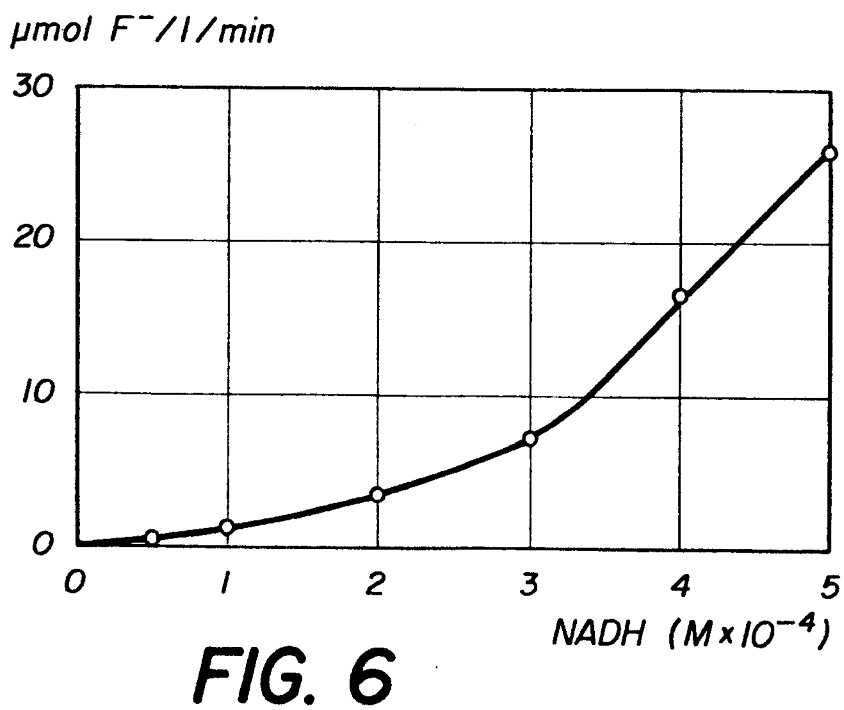
µmol F⁻/l/min
30
20
10
0
0
1
2
3
4
5
NADH (M×10⁻⁴)

FIG. 6